# EUROPEAN PATENT SPECIFICATION

(11) **EP 0 410 422 B1**
(45) Date of publication and mention of the grant of the patent: **02.03.1994**
(21) Application number: 90114268.7
(22) Date of filing: 25.07.1990
(51) Int. Cl.: A61K 31/44, A61K 9/18

(54) **A highly absorbable pharmaceutical composition**
Stark absorbierbares Arzneimittel
Composition pharmaceutique hautement absorbable

(30) Priority: 25.07.1989 JP 192413/89
(43) Date of publication of application: 30.01.1991
(73) Proprietor: OTSUKA PHARMACEUTICAL CO., LTD., Chiyoda-ku Tokyo (JP)
(72) Inventor: Ishizue, Yoshihiro, Itano-gun, Tokushima-ken (JP); Ishida, Kozo, Itano-gun, Tokushima-ken (JP); Odomi, Masaaki, Tokushima-shi (JP); Nishibayashi, Toru, Tokushima-shi (JP); Koshino, Kazuo, Tokushima-shi (JP)
(74) Representative: Hansen, Bernd, Dr. Dipl.-Chem.

(56) References cited:
- EP-A- 0 231 026

## Description

The present invention relates to a highly absorbable pharmaceutical composition.

Methyl 3-phenyl-2(E)-propenyl 1,4-dihydro-2,6-dimethyl-4-(3-nitrophenyl)pyridine-3,5-dicarboxylate (hereinafter referred to as "compound of the present invention"), has effective pharmacological activities for example antihypertensive effect and others, which has been developed by the present inventors [Japanese Patent Kokai (Laid-open) No. 60-120861 (1985), and European Patent Publication No. 0,145,434 (June 19, 1985)]. However, the compound of the present invention is practically insoluble in an aqueous solution (0.5 µg/ml), and has quite low absorbability through the gastrointestinal tract when it is administered orally.

EP-0 231 026 discloses a pharmaceutical composition including a dihydropyridine calcium channel blocker which is prepared by forming an essentially aqueous wet mass comprising the above active ingredient with a pH-dependent binder which may be represented by a copolymer of methacrylic acid and a methacrylic or acrylic acid ester, extruding the wet mass to form rod-shaped, substantially cylindrical segments, shaping the rod-shaped segments into spheroids and drying the spheroids.

The present inventors have made an extensive study for improving the solubility of compound of the present invention in an aqueous solution, so as to make it possible to sustain a supersaturated state thereof for a long period of time, and to improve the absorbability thereof through the gastrointestinal tract. During the course of study, the present inventors have tried to improve the solubility thereof in an aqueous solution by making it as an amorphous state with various types of polymers. However, in the case of preparing the amorphous state by using polymers such as a cellulose-type high molecular compound or a polyvinylpyrrolidone or the like, the solubility of compound of the present invention could not have been improved as expected. Under the circumstances, the present inventors further have continued the study and finally have found the fact that the object of the present invention can be achieved only if the compound of the present invention were made as in the form of a amorphous state by using pH-dependent type methacrylic acid and its derivatives copolymers. The present invention, thus has successfully been completed on the basis of such findings.

An object of the present invention is to provide a highly absorbable pharmaceutical composition wherein the composition contains an amorphous state consisting of methyl 3-phenyl-2(E)-propenyl 1,4-dihydro-2,6-dimethyl-4-(3-nitrophenyl)-pyridine-3,5-dicarboxylate and pH-dependent type methacrylic acid copolymers.

The above problems is solved by a highly absorbable pharmaceutical composition obtainable by mixing together in an organic solvent, as to the first component, methyl 3-phenyl-2(E)-propenyl 1,4-dihydro-2,6-dimethyl-4-(3-nitrophenyl)pyridine-3,5-dicarboxylate and as to the second component thereof, at least one pH-dependent copolymer of methacrylic acid selected from the group consisting of pH-dependent trimethylammonium salts of copolymers of acrylic acid and methacrylic acid; pH-dependent coploymers of methacrylic acid and methyl acrylate; pH-dependent copolymers of methacrylic acid and methyl methacrylate; pH-dependent copolymers of ethyl acrylate and methyl methacrylate; and pH-dependent copolymers of butyl methacrylate, dimethylaminoethyl methacrylate and methyl methacrylate, said organic solvent being selected from the group consisting of acetone, methanol, ethanol, isopropanol, methylene chloride, chloroform and mixtures thereof and being present in an amount sufficient to completely dissolve both of said components, wherein the ratio of the amount of first component to the amount of the second component is from 10 to 2,000 parts by weight of the second component per 100 parts by weight of the first component, and thereafter by removing the solvent to obtain said composition, wherein the first component is contained as in the form of an amorphous state.

According to a preferred embodiment, the ratio of the first component to the second component is from 30 to 1,000 parts by weight of the second component per 100 parts by weight of the first component.

In further preferred embodiment, the solvent is a 3:7 mixture of ethanol and methylene chloride.

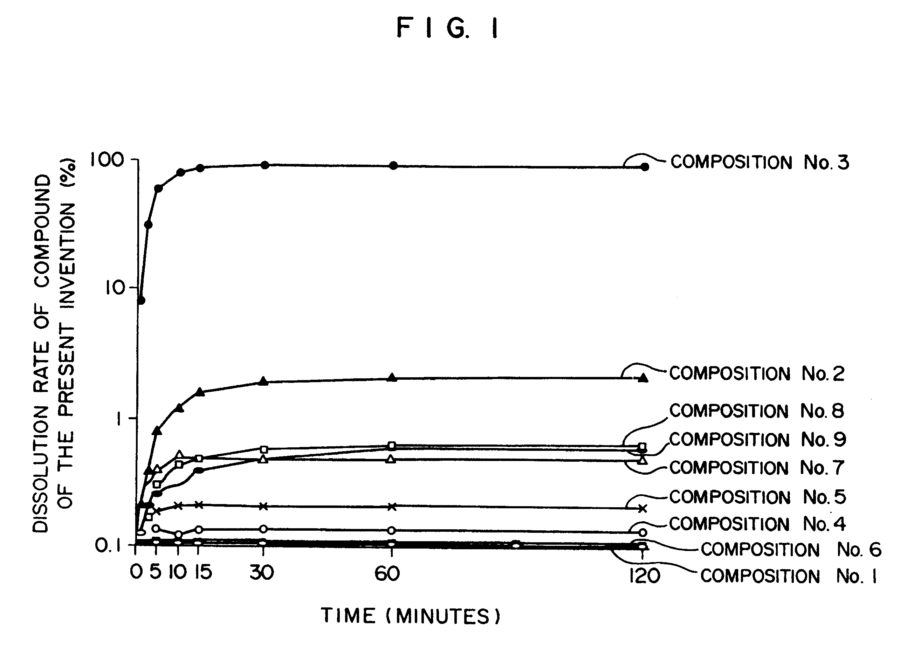

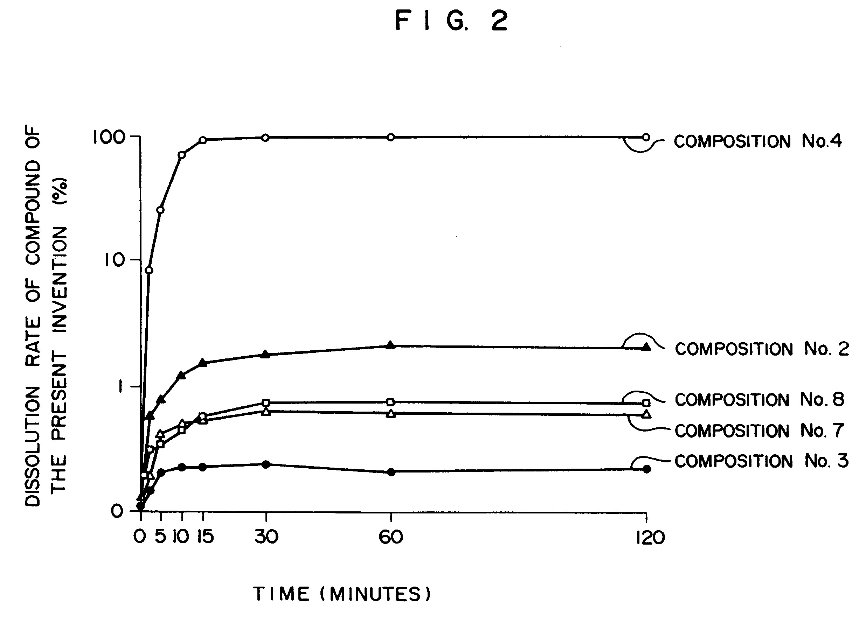

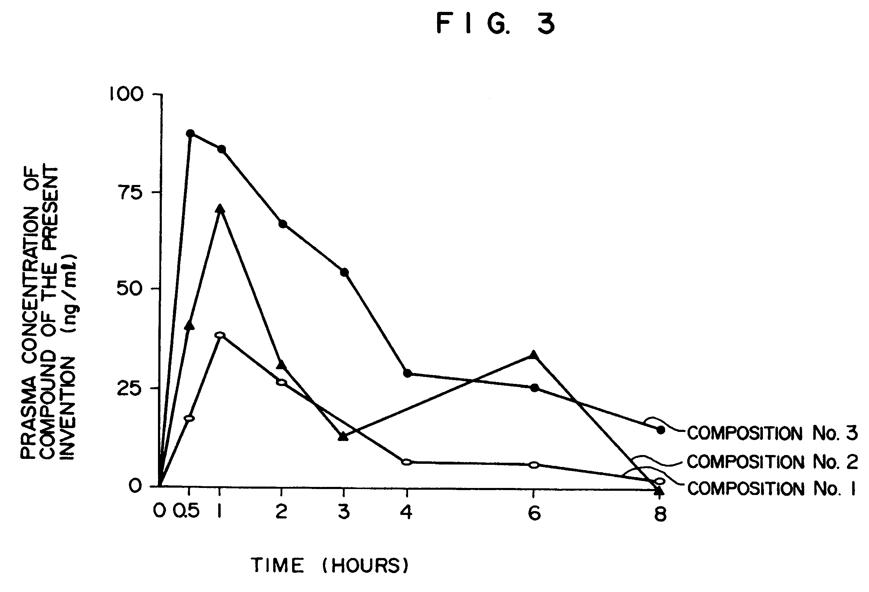

Fig. 1 and Fig. 2 show curves indicating the time-sequential changes of dissolution rate (%) of compound of the present invention dissolved from various compositions of the present invention. Fig. 3 shows curves indicating the time-sequential changes of plasma concentration (ng/ml) of compound of the present invention, released from various compositions of the preesent invention, after the oral administration in begle dogs.

As to the pH-dependent type methacrylic acid and its derivatives copolymers, various methacrylic acid and its derivatives type coplomers which are widely known in the art can be employed as far as they have the pH-dependency, and the the examples are including such as trimethylammonium salts of copolymers of acrylic acid and methacrylic acid; copolymers of methacrylic acid and methyl acrylate; copolymers of methacrylic acid and methyl metharylate; copolymers of ethyl acrylate and methyl methacrylate, copolymers of butyl methacrylate, dimethylaminoethyl methacrylate and methyl methacrylate.

More specifically, Eudragit® L, Eudragit® S, Eudragit® E, Eudragit® L100, Eudragit® S100 and Eudragit® E100 (they are trademarks for pH-dependent type methacrylic acid and derivatives copolymers manufactured by Röhm and Pharma GmbH) can be exemplified.

Among these pH-dependent type metharylic acid and its derivatives copolymers, Eudragit® E is an example of an aminoalkyl methacrylate copolymer which is soluble in the gastric juice, and Eudragit® L and Eudragit® S are examples of methacrylic acid copolymers which are soluble in the intestinal juice. In the highly absorbable pharmaceutical compositions of the present invention, the above-mentioned pH-dependent type methacrylic acid and its derivatives copolymers can be used alone or in combination of two or more thereof.

As to the mixed ratio of the amount of compound of the present invention to the amount of the pH-dependent type methacrylic acid and its derivatives copolymers, generally 10 to 2,000 parts by weight, preferably 30 to 1,000 parts by weight of the latter may be used to 100 part by weight of the former.

If necessary, various kinds of additives, for example, water-soluble or water-swelling polymers, surfactants, polyethylene glycols, organic acids and the like which may usually be formulated with this type of pharmaceutical compositions, can also suitably be formulated with the composition of the present invention.

As to the water-soluble or water-swelling polymers, the examples are including, hydroxypropylmethyl cellulose, hydroxypropyl cellulose, hydroxyethyl cellulose, polyvinylpyrrolidone, pH-independent methacrylic acid and its derivatives copolymers (e.g., Eudragit® RL Eudragit® RS,)
As to the surfactants, non-ionic, cationic, anionic, and amphoteric surfactants can be exemplified.

Furthermore, as to the organic acids, the examples are including citric acid, succinic acid and tartaric acid.

The highly absorbable antihypertensive composition according to the present invention can be prepared by the methods as mentioned below, thus compound of the present invention, pH-dependent type methacrylic acid and its derivatives copolymers, additionally, if necessary, water-soluble or water-swelling polymers, surfactants, polyethylene glycols and organic acids are dissolved in an organic solvent, then the organic solvent is removed by evaporation by conventional methods. In this case, organic acids and other excipients for tablet compression may also be added to the composition, or an organic acid and other excipients for tablet compression may be added previously, then the organic solvent may be removed by evaporation.

As to the excipients for tablet compression, any excipients which are known widely in the art may be employed.

The organic solvent used according to the present invention must be able to dissolve both the compound of the present invention and the pH-dependent type methacrylic acid and its derivative copolymers. The examples of the solvents include acetone, methanol, ethanol, isopropanol, dichloromethane and chloroform. Furthermore, if necessary, water may be added as a solvent.

As to the methods for removing the organic solvents, evaporation, spray drying, fluidized bed coating, centrifugal fluidized bed coating methods can be employed.

### EFFECTS OF THE INVENTION

The composition of the present invention makes it possible to improve the solubility of the compound of the present invention in an aqueous solution, it makes it possible to keep a supersaturated state thereof in an aqueous solution for a long period of time, further it improves the absorbability thereof through the gastrointestinal tract when the composition is administered orally.

The compound of the present invention has been developed as mainly for anti-hypertensive agent, and as to pharmaceutical compositions, containing thereof as the active ingredient, which can be able to sustain its pharmacological activities for a long period of time, with less side-effects has eagerly been expected.

There has been indeed known the facts in that when the compound of the present invention was dissolved in a solvent such as a polyethylene glycol then thus obtained solution is orally administered, the absorbability of the compound of the present invention can be improved in certain extent. However, the absorbability of the compound of the present invention cannot suitably be controlled when such a solution-type pharmaceutical composition is orally administered, there has been worried about in that any side-effect would be appeared due to a rapid increasing of the plasma concentration of the compound of the present invention.

According to the present invention, when the highly absorbable pharmaceutical composition is administered orally, the compound of the present invention which is contained as the active ingredient in the composition, and which could not have easily been absorbed through the gastrointestinal tract, can be absorbed sufficiently. In addition to the above, by selecting type of polymer materials such as the pH-dependent type methacrylic acid and its derivative copolymers and the ratio of the amount thereof to the amount of compound of the present invention in the composition, the side-effects which may possibly be caused by a rapid increasing of the plasma concentration of compound of the present invention can easily be controlled.

### EXAMPLES

The present invention will be further explained by illustrating the following Examples and Examples of pharmaceutical compositions.

### Example 1

Into a fluidized bed granularing machine, 228 g of lactose and 30 g of maize starch were placed, then by using 300 g of 4%-aqueous solution of hydroxypropylmethyl cellulose as a binding agent to make the whole mixture to granules. Separately, 9 g of compound of the present invention and 27 g of each one of polymers as mentioned below were dissolved completely in a mixed solvent of methanol-methylene chloride (1:1), respectively. Then, each one of said solutions was coated by spraying on the granules to make the following 8 types of pharmaceutical compositions, respectively. Thus prepared coated granules of each one of the compositions were filled in capsules, separately.

### 1) Pharmaceutical composition No. 3

Eudragit® L100 (trademark for a methacrylic acid copolymer L manufactured by Röhm and Pharma GmbH) was used as the polymer in the pharmaceutical composition.

### 2) Pharmaceutical composition No. 4

Eudragit® E100 (trademark for a aminoalkyl methacrylate copolymer manufactured by Röhm and Pharma GmbH) was used as the polymer in the pharmaceutical composition.

### 3) Pharmaceutical composition No. 5

Eudragit® RL100 (trademark for a aminoalkyl methacrylate copolymer E manufactured by Röhm and Pharma GmbH) was used as the polymer in the pharmaceutical composition.

### 4) Pharmaceutical composition No. 6

Eudragit® RS100 (trademark for a aminoalkyl methacrylate copolymer RS manufactured by Röhm and Pharma GmbH) was used as the polymer in the pharmaceutical composition.

### 5) Pharmaceutical composition No. 2

Plasdone® K-29/32 (trademark for a polyvinylpyrrolidone manufactured by GAF Chemical Corp.) was used as the polymer in the pharmaceutical composition.

### 6) Pharmaceutical composition No. 7

TC-5E® (trademark for a hydroxypropylmethyl cellulose manufactured by Shin-Etsu Chemical Co., Ltd.) was used as the polymer in the pharmaceutical composition.

### 7) Pharmaceutical composition No. 8

HPC-L® (trademark for a hydroxypropylmethyl cellulose manufactured by Nippon Soda Co., Ltd.) was used as the polymer in the pharmaceutical composition.

### 8) Pharmaceutical composition No. 9

HP-55® (trademark for a hydroxypropylmethyl cellulose phthalate manufactured by Shin-Etsu Chemical Co., Ltd.) was used as the polymer in the pharmaceutical composition.

### 9) Pharmaceutical composition No. 1 (Reference Composition)

Into a fluidized bed granulating machine, 249 g of lactose, 30 g maize starch and 9 g of compound of the present invention were placed, then by using 4%-aqueous solution of hydroxypropylmethyl cellulose as a binding agent to make the whole mixture to granules, which are referred to as Reference Composition.

### Example 2

To 300 g each of pharmaceutical composition Nos. 3 and 4 prepared in Example 1, were added 50 g of crystalline cellulose, 100 g of lactose, 20 g of croscarmellose sodium and 5 g of magnesium stearate as the excipients for tablet compression, and the whole mixture was compressed to make the pharmaceutical composition of the present invention as tables.

### Example 3

By using 200 g of pharmaceutical composition No. 3 and 100 g of Pharmaceutical Composition No. 4 prepared in Example 1 and they were mixed previously, then said mixture was further added with the excipients for tablet compression used in Example 2, and the whole mixture was compressed to make the pharmaceutical composition of the present invention as tables.

### Example 4

Nine grams of compound of the present invention, 9 g of Eudragit® L100 (trademark of a methacrylic acid copolymer manufactured by Röhm and Pharma GmbH), 3 g of Plasdone® K-29/32 (trademark for a polyvinyl pyrrolidone manufacture by GAF Chemical Corp.), 0.1 g of Tween® 80 (trademark for a surfactant manufactured by Kao-Atlas Corp.) and 0.5 g of polyethylene glycol 6000 were dissolved completely in a mixed solvent of ethanol-chloroform (1:1), then this solution was concentrated to dryness by an evaporation method and thus obtained solid mass was pulverized into powders having suitable particle size. To said powders were added 130 g of lactose, 60 g of crystalline cellulose and 1 g of magnesium stearate and the whole mixture was compressed to make the pharmaceutical composition of the present invention as tablets.

### Example 5

Ninety grams of compound of the present invention, 9 g of Eudragit® E100 (trademark for a pH-dependent aminoalkyl methacrylate copolymer E manufactured by Röhm and Pharma GmbH) and 3 g of TC-5E® (trademark for a hydroxypropylmethyl cellulose manufactured by Shin-Etsu Chemical Co., Ltd.) were dissolved completely in a mixed solvent of ethanol-methylene chloride (1:1), then this solution was subjected to spray-drying to make it in the form of powder. Then this powder was made into granules by using a 4%-aqueous solution of hydroxypropylmethyl cellulose, containing 2 g of citric acid and dried. Next, to the dried granules were added 1,200 g of lactose, 400 g of crystalline cellulose and 10 g of magnesium stearate, and the whole mixture was subjected to tablet compression to obtain the pharmaceutical composition.

### Example 6

Ten grams of compound of the present invention and 5 g of Eudragit® L (trademark for a methacrylic acid copolymer L manufactured by Röhm and Pharma GmbH) were dissolved in 125 g of a mixed solvent of methylene chloride-ethanol (75:25), then this solution was subjected to spray-drying at 70°C to make it in the form of powder. To 15 g of this powder were added 285 g of lactose, 125 g of crystalline cellulose, 7.5 g of croscarmellose sodium and 2.5 g of magnesium stearate to make the whole mixture as a composition for compression molding. Said composition was then subjected to tablet compression under a tableting pressure at 400 kg. Each tablets thus obtained contains 87 mg of compound of the present invention.

### Example 7

Nine grams of compound of the present invention, 90 g of Eudragit® L100 (trademark for a methacrylic acid copolymer L manufactured by Röhm and Pharma GmbH), 3 g of Plasmidone K-29/32® (trademark for a polyvinylpyrrolidone manufactured by GAF Chemical Corp.), 0.1 g of Tween® 80 (trademark for a surfactant manufactured by Kao-Atlas Corp.) and 0.5 g of polyethylene glycol 6000 were dissolved completely in a mixed solvent of ethanol-chloroform (1:1), then this solution was concentrated to dryness by an evaporation and thus obtained solid mass was pulverized into powders having suitable particle size. To said powders were added 130 g of lactose, 60 g of crystalline cellulose and 1 g of magnesium stearate to make the whole mixture as a pharmaceutical composition. Said composition was filled in capsules.

### Example 8

Ninety grams of compound of the present invention, 27 g of Eudragit® E100 (trademark for a aminoalkyl methacrylate copolymer E manufactured by Röhm and Pharma GmbH) and 3 g of TC-5E® (trademark for a hydroxymethyl cellulose manufactured by shin-Etsu Chemical Co., Ltd.) were dissolved completely in a mixed solvent of ethanol-methylene chloride (1:1), then this solution was subjected to spray-drying to make it in powder form. This powder form product was then subjected to granulation by using a 4%-aqueous solution of hydroxypropylmethyl cellulose containing, 2 g of citric acid, as a binding agent, and was dried. To thus obtained dried granules were added 1,200 g of lactose, 400 g of crystalline cellulose and 10 g of magnesium stearate, then the whole mixture thus obtained was subjected to compression molding to make it in the form of tablets of pharmaceutical composition of the present invention.

### Example 9

Ninety grams of compound of the present invention and 63 g of Eudragit® L100 (trademark for a methacrylic acid copolymer L manufactured by Röhm and Pharma GmbH) were dissolved completely in a mixed solvent of ethanol-methylene chloride (3:7), then this solution was subjected to spray-drying to make it in powder form. This powder form products was pulverized by using a jet-mill to obtain microfine powder products. To 17 g of this powder products were added 600 g of lactose, 260 g of crystalline cellulose, and 15 g of croscarmellose sodium then they were mixed thoroughly, further 5 g of magnesium stearate was added thereto as a lubricant, and make the whole mixture as a composition for compression molding, then this composition was made into tablets.

### Dissolution Test

The test was conducted by a method and apparatus according to Dissolution Test Method No. 2 (Paddle Method) being provided in Japanese Pharmacopoeia [XIth Revised Edition (1986)]. One hundred (100) microliters each of test solutions was sampled in time-sequential manner (5, 10, 15, 30, 60 and 120 minutes, respectively) by using a microsyringe, and the amount of compound of the present invention being dissolved from each of the pharmaceutical compositions of the present invention being tested was assayed by use of a high performance liquid phase chromatography.

As to the eluants, the First Fluid (pH 1.2) and the Second Fluid (pH 6.8) which are provided in Japanese Pharmacopoeia (XIth Revised Edition) were used.

Nine-hundreds ml of each of the test solutions (First and Second Fluids) was placed in the vessel, then the paddle was rotated at a rate of 150 rpm, under a temperature of at 37.0 ± 0.5°C. Then, each one of the pharmaceutical compositions, containing 10 mg of compound of the present invention, as the test sample was put into the vessel. The test solution was sampled in time sequential manner and the amount of compound of the present invention being dissolved from the test composition sample was detected by calculating the peak area at 245 nm by a high performance liquid phase chromatography and assayed by an absolute calibration curve method.

Fig. 1 shows curves indicating the time sequential changes of dissolution rate (%) of pharmaceutical compositions of the present invention (Compositions Nos. 1-9) prepared in Example 1, and the test was conducted by using the Second Fluid (pH 6.8), and Fig. 2 shows curves indicating the time sequential changes of dissolution rate (%) of pharmaceutical compositions of the present invention (Compositions Nos. 2, 3, 4, 7 and 8) prepared in Example 1, and the test was conducted by using the First Fluid (pH 1.2).

As can clearly been seen from Fig. 1, that in case of conducting the test by using the Second Fluid (pH 6.8), the test sample of pharmaceutical Composition No. 3, in which Eudragit® L100 was used, performs specifically higher improvement of dissolution rate as compared with those of performed by other compositions (Composition Nos. 1, 2 and 4-9), furthermore, Composition No. 3 can be able to sustain the supersaturation state of compound of the present invention for a long period of time.

As can be seen from Fig. 2, that in case of conducting the test by using the First Fluid (pH 1.2), the test sample of pharmaceutical Composition No. 4, in which Eudragit® E100 was used, performs specifically higher improvement of dissolution rate as compared with those of performed by other compositions (Composition Nos. 2, 3, 7 and 8).

There can be known from these test results in that, the dissolution rate of compound of the present invention can be improved considerably when compound of the present invention is formulated with Eudragit® E100 and the formulated composition is used in an acidic region. While, the elution rate of compound of the present invention can be improved considerably when compound of the present invention is formulated with Eudragit® L100 and the formulated composition is used in neutral and basic regions.

Therefore, pharmaceutical compositions of the present invention can preferably be prepared by using singly or both of formulating ingredients, each of which contains either one of the above-mentioned two types of polymers, respectively. Furthermore, in addition to the above-mentioned two types of polymers, other types of polymers, surfactants, polyethylene glycols, organic acids as to the third member for the formulating ingredient may be added singly or both members with the formulating ingredients, as well as by adding suitable excipients for compression molding and then the whole mixture is made into the desired form.

The amount of compound of the present invention dissolved from the pharmaceutical composition of the present invention can be controlled by changing basically the ratio of the amount of Eudragit L100 or Eudragit® E100 to the amount of the compound of the present invention, and/or by changing the tablet compression conditions.

### Comparative Absorption Test

### (1) Test animal

Five (5) beagle dogs (having 10 to 12 kg of body weight) were fasted overnight and were used as the test animals.

### (2) Administration method

Five (5) test dogs were kept away from oral administration of test composition for over one week, then each one of the pharmaceutical compositions (Composition Nos. 1-3) was orally administered to the test dog, at a dose of 30 mg of compound of the present invention per dog.

### (3) Sampling the blood

About 3 ml of the blood was sampled from the test dog in time-sequentially (0.5, 1, 2, 3, 4, 6 and 8 hours) after the oral administration by using a syringe treated with hepaline. The blood sample was centrifuged at 3,000 rpm to obtain the serum, and said serum was freezed at -20°C and stored until its use for the measurement of concentration of compound of the present invention.

The amount of compound of the present invention in the serum sample was assayed by means of a high performance liquid phase chromatography. The test results are shown in Fig. 3.

As can be seen from data shown in Fig. 3, in that compound of the present invention was rapidly increased in its plasma concentration in the pharmaceutical composition (Composition No. 3) using Eudragit® L100 as the polymer, and was well absorbed from the gastrointestinal tract, and it could be able to keep the effective blood concentration of compound of the present invention for a long period of time, as compared with those of other compositions (Composition Nos. 1 and 2).

## Claims (Claims for the following Contracting State(s): CH, DE, DK, FR, GB, IT, LI, NL, SE)

1. A highly absorbable pharmaceutical composition obtainable by mixing together in an organic solvent, as to the first component thereof, methyl 3-phenyl-2-(E)-propenyl 1,4-dihydro-2,6-dimethyl-4-(3-nitrophenyl)pyridine-3,5-dicarboxylate and, as to the second component thereof, at least one pH-dependent copolymer of methacrylic acid selected from pH-dependent trimethylammonium salts of copolymers of acrylic acid and methacrylic acid; pH-dependent copolymers of methacrylic acid and methyl acrylate; pH-dependent copolymers of methacrylic acid and methyl methacrylate; pH-dependent copolymers of ethyl acrylate and methyl methacrylate; and pH-dependent copolymers of butyl methacrylate, dimethylaminoethyl methacrylate and methyl methacrylate, said organic solvent being selected from acetone, methanol, ethanol, isopropanol, methylene chloride, chloroform and mixtures thereof and being present in an amount sufficient to completely dissolve both of said first and second components, wherein the ratio of the amount of the first component to the amount of the second component is from 10 to 2,000 parts by weight of the second component per 100 parts by weight of the first component, and thereafter by removing the solvent to obtain said composition, wherein the first component is contained as in the form of an amorphous state.

2. The composition according to Claim 1, wherein the ratio of the amount of the first component to the amount of the second component is from 30 to 1,000 parts by weight of the second component per 100 parts by weight of the first component.

3. The composition according to Claim 1, wherein the solvent is a 3:7 mixture of ethanol and methylene chloride.

4. A process for preparing highly absorbable pharmaceutical composition of methyl 3-phenyl-2(E)-propenyl 1,4-dihydro-2,6-dimethyl-4-(3-nitrophenyl)pyridine-3,5-dicarboxylate as to the first component thereof and, as to the second component thereof, a pH-dependent copolymer of methacrylic acid selected from pH-dependent trimethylammonium salts of copolymers of acrylic acid and methacrylic acid; pH-dependent copolymers of methacrylic acid and methyl acrylate; pH-dependent copolymers of methacrylic acid and methyl methacrylate; pH-dependent copolymers of ethyl acrylate and methyl methacrylate; and pH-dependent copolymers of butyl methacrylate, dimethylaminoethyl methacrylate and methyl methacrylate, which comprises:
(i) by mixing said components together in an organic solvent selected from acetone, methanol, ethanol, isopropanol, methylene chloride, chloroform and mixture thereof, wherein said solvent is present in an amount sufficient to completely dissolve both of said first and second components and the ratio of the amount of the first component to the amount of the second component is from 10 to 2,000 parts by weight of the second component per 100 parts by weight of the first component, and
(ii) thereafter by removing said solvent to obtain said composition, wherein the first component is contained as in the form of an amorphous state.

5. The process according to Claim 4, wherein the ratio of the amount of the first component to the amount of the second component is from 30 to 1,000 parts by weight of the second component per 100 parts by weight of the first component.

6. The process according to Claim 4, wherein the solvent is a 3:7 mixture of ethanol and methylene chloride.

7. Use of a pharmaceutical composition according to Claims 1 to 3 for preparing a medicament.

8. Use of a pharmaceutical composition according to Claims 1 to 3 for preparing an antihypertensive agent.

## Claims (Claims for the following Contracting State(s): ES)

1. A process for preparing highly absorbable pharmaceutical composition of methyl 3-phenyl-2(E)-propenyl 1,4-dihydro-2,6-dimethyl-4-(3-nitrophenyl)pyridine-3,5-dicarboxylate as to the first component thereof and, as to the second component thereof, a pH-dependent copolymer of methacrylic acid selected from pH-dependent trimethylammonium salts of copolymers of acrylic acid and methacrylic acid; pH-dependent copolymers of methacrylic acid and methyl acrylate; pH-dependent copolymers of methacrylic acid and methyl methacrylate; pH-dependent copolymers of ethyl acrylate and methyl methacrylate; and pH-dependent copolymers of butyl methacrylate, dimethylaminoethyl methacrylate and methyl methacrylate, which comprises (i) by mixing said components together in an organic solvent selected from acetone, methanol, ethanol, isopropanol, methylene chloride, chloroform and mixture thereof, wherein said solvent is present in an amount sufficient to completely dissolve both of said components and the ratio of the amount of the first component to the amount of the second component is from 10 to 2,000 parts by weight of the second component per 100 parts by weight of the first component, and (ii) thereafter by removing said solvent to obtain said composition, wherein the first component is contained as in the form of an amorphous state.

2. The process according to Claim 1, wherein the ratio of the amount of the first component to the amount of the second component is from 30 to 1,000 parts by weight of the second component per 100 parts by weight of the first component.

3. The process according to Claim 1, wherein the solvent is a 3:7 mixture of ethanol and methylene chloride.

4. Use of a pharmaceutical composition according to Claims 1 to 3 for preparing a medicament.

5. Use of a pharmaceutical composition according to Claims 1 to 3 for preparing an antihypertensive agent.

## Patentansprüche (Patentansprüche für folgende(n) Vertragsstaat(en): CH, DE, DK, FR, GB, IT, LI, NL, SE)

1. Stark absorbierbare, pharmazeutische Zusammensetzung, erhältlich durch Mischen von Methyl-3-phenyl-2(E)-propenyl-1,4-dihydro-2,6-dimethyl-4-(3-nitrophenyl)pyridin-3,5-dicarboxylat als erste Komponente und mindestens eines pH-Wert-abhängigen Copolymers von Methacrylsäure als zweite Komponente, ausgewählt aus pH-Wert-abhängigen Trimethylammoniumsalzen von Copolymeren von Acrylsäure und Methacrylsäure; pH-Wert-abhängigen Copolymeren von Methacrylsäure und Methylacrylat; pH-Wert-abhängigen Copolymeren von Methacrylsäure und Methylmethacrylat; pH-Wert-abhängigen Copolymeren von Ethylacrylat und Methylmethacrylat; und pH-Wert-abhängigen Copolymeren von Methylmethacrylat, Dimethylaminoethylmethacrylat und Methylmethacrylat, in einem organischen Lösungsmittel, wobei das organische Lösungsmittel aus Aceton, Methanol, Ethanol, Isopropanol, Methylenchlorid, Chloroform und Mischungen hiervon ausgewählt ist und in einer Menge vorliegt, die ausreicht, um die ersten und zweiten Komponenten vollständig zu lösen, wobei das Verhältnis der Menge der ersten Komponente zu der Menge der zweiten Komponente bei 10 bis 2.000 Gewichtsteilen der zweiten Komponente pro 100 Gewichtsteile der ersten Komponente liegt, und anschließendes Entfernen des Lösungsmittels, um die Zusammensetzung zu erhalten, wobei die erste Komponente in Form eines amorphen Zustands enthalten ist.

2. Zusammensetzung nach Anspruch 1, wobei das Verhältnis der Menge der ersten Komponente zu der Menge der zweiten Komponente bei 30 bis 1.000 Gewichtsteilen der zweiten Komponente pro 100 Gewichtsteile der ersten Komponente liegt.

3. Zusammensetzung nach Anspruch 1, wobei das Lösungsmittel eine 3:7-Mischung von Ethanol und Methylenchlorid ist.

4. Verfahren zur Herstellung einer stark absorbierbaren, pharmazeutischen Zusammensetzung aus Methyl-3-phenyl-2(E)-propenyl-1,4-dihydro-2,6-dimethyl-4-(3-nitrophenyl)pyridin-3,5-dicarboxylat als erste Komponente und einem pH-Wert-abhängigen Copolymer von Methacrylsäure als zweite Komponente, ausgewählt aus pH-Wert-abhängigen Trimethylammoniumsalzen von Copolymeren von Acrylsäure und Methacrylsäure; pH-Wert-abhängigen Copolymeren von Methacrylsäure und Methylacrylat; pH-Wert-abhängigen Copolymeren von Methacrylsäure und Methylmethacrylat; pH-Wert-abhängigen Copolymeren von Ethylacrylat und Methylmethacrylat; und pH-Wert-abhängigen Copolymeren von Butylmethacrylat, Dimethylaminoethylmethacrylat und Methylmethacrylat, das die Schritte umfaßt:
(i) Vermischen der Komponenten in einem organischen Lösungsmittel, ausgewählt aus Aceton, Methanol, Ethanol, Isopropanol, Methylenchlorid, Chloroform und Mischungen hiervon, wobei das Lösungsmittel in einer Menge vorliegt, die ausreicht, um die ersten und zweiten Komponenten vollständig zu lösen, und wobei das Verhältnis der Menge der ersten Komponente zu der Menge der zweiten Komponente im Bereich von 10 bis 2.000 Gewichtsteilen der zweiten Komponente pro 100 Gewichtsteile der ersten Komponente liegt, und
(ii) anschließendes Entfernen des Lösungsmittels, um die Zusammensetzung zu erhalten, wobei die erste Komponente in der Form eines amorphen Zustandes enthalten ist.

5. Verfahren nach Anspruch 4, wobei das Verhältnis der Menge der ersten Komponente zu der Menge der zweiten Komponente im Bereich von 30 bis 1.000 Gewichtsteilen der zweiten Komponente pro 100 Gewichtsteile der ersten Komponente liegt.

6. Verfahren nach Anspruch 4, wobei das Lösungsmittel eine 3:7-Mischung von Ethanol und Methylenchlorid ist.

7. Verwendung einer pharmazeutischen Zusammensetzung gemäß den Ansprüchen 1 bis 3 zur Herstellung eines Arzneimittels.

8. Verwendung einer pharmazeutischen Zusammensetzung gemaß den Ansprüchen 1 bis 3 zur Herstellung eines blutdrucksenkenden Mittels.

## Patentansprüche (Patentansprüche für folgende(n) Vertragsstaat(en): ES)

1. Verfahren zur Herstellung einer stark absorbierbaren, pharmazeutischen Zusammensetzung aus Methyl-3-phenyl-2(E)-propenyl-1,4-dihydro-2,6-dimethyl-4-(3-nitrophenyl)pyridin-3,5-dicarboxylat als erste Komponente und einem pH-Wert-abhängigen Copolymer von Methacrylsäure als zweite Komponente, ausgewählt aus pH-Wert-abhängigen Trimethylammoniumsalzen von Copolymeren von Acrylsäure und Methacrylsäure; pH-Wert-abhängigen Copolymeren von Methacrylsäure und Methylacrylat; pH-Wert-abhängigen Copolymeren von Methacrylsäure und Methylmethacrylat; pH-Wert-abhängigen Copolymeren von Ethylacrylat und Methylmethacrylat; und pH-Wert-abhängigen Copolymeren von Butylmethacrylat, Dimethylaminoethylmethacrylat und Methylmethacrylat, das die Schritte umfaßt:
(i) Vermischen der Komponenten in einem organischen Lösungsmittel, ausgewählt aus Aceton, Methanol, Ethanol, Isopropanol, Methylenchlorid, Chloroform und Mischungen hiervon, wobei das Lösungsmittel in einer Menge vorliegt, die ausreicht, um die ersten und zweiten Komponenten vollständig zu lösen, und wobei das Verhältnis der Menge der ersten Komponente zu der Menge der zweiten Komponente im Bereich von 10 bis 2.000 Gewichtsteilen der zweiten Komponente pro 100 Gewichtsteile der ersten Komponente liegt, und
(ii) anschließendes Entfernen des Lösungsmittels, um die Zusammensetzung zu erhalten, wobei die erste Komponente in der Form eines amorphen Zustandes enthalten ist.

2. Verfahren nach Anspruch 1, wobei das Verhältnis der Menge der ersten Komponente zu der Menge der zweiten Komponente im Bereich von 30 bis 1.000 Gewichtsteilen der zweiten Komponente pro 100 Gewichtsteile der ersten Komponente liegt.

3. Verfahren nach Anspruch 1, wobei das Lösungsmittel eine 3:7-Mischung von Ethanol und Methylenchlorid ist.

4. Verwendung einer pharmazeutischen Zusammensetzung gemäß den Ansprüchen 1 bis 3 zur Herstellung eines Arzneimittels.

5. Verwendung einer pharmazeutischen Zusammensetzung gemäß den Ansprüchen 1 bis 3 zur Herstellung eines blutdrucksenkenden Mittels.

## Revendications (Revendications pour l'(les) Etat(s) contractant(s) suivant(s): CH, DE, DK, FR, GB, IT, LI, NL, SE)

1. Composition pharmaceutique hautement absorbable susceptible d'être obtenue en mélangeant ensemble dans un solvant organique, en tant que son premier composant, du 1,4-dihydro-2,6-dimethyl-4-(3-nitrophényl)pyridine-3,5-dicarboxylate de méthyle et de 3-phényl-2-(E)-propényle et, en tant que son deuxième composant, au moins un copolymère dépendant du pH d'acide méthacrylique, choisi parmi les sels de triméthylammonium dépendants du pH de copolymères d'acide acrylique et d'acide méthacrylique ; les copolymères dépendants du pH d'acide méthacrylique et d'acrylate de méthyle ; les copolymères dépendants du pH d'acide méthacrylique et de méthacrylate de méthyle ; les copolymères dépendants du pH d'acrylate d'éthyle et de méthacrylate de méthyle ; et les copolymères dépendants du pH de méthacrylate de butyle, de méthacrylate de diméthylaminoéthyle et de méthacrylate de méthyle, le solvant organique étant choisi parmi l'acétone, le méthanol, l'éthanol, l'isopropanol, le chlorure de méthylène, le chloroforme et les mélanges de ceux-ci, et il est présent selon une quantité suffisante pour dissoudre complètement tant le premier que le deuxième composants, le rapport de la quantité du premier composant à la quantité du deuxième composant, étant de 10 à 2 000 parties en poids du deuxième composant pour 100 parties en poids du premier composant, et en éliminant ensuite le solvant pour obtenir ladite composition, le premier composant étant contenu sous une forme à l'état amorphe.

2. Composition selon la revendication 1, dans laquelle le rapport de la quantité du premier composant à la quantité du deuxième composant, est de 30 à 1 000 parties en poids du deuxième composant pour 100 parties en poids du premier composant .

3. Composition selon la revendication 1, dans laquelle le solvant est un mélange 3:7 d'éthanol et de chlorure de méthylène.

4. Procédé de préparation d'une composition pharmaceutique hautement absorbable comprenant du 1,4-dihydro-2,6-diméthyl-4-(3-nitrophényl)pyridine-3,5-dicarboxylate de méthyle et de 3-phényl-2-(E)-propényle en tant que son premier composant, et en tant que son deuxième composant, un copolymère dépendant du pH d'acide méthacrylique choisi parmi les sels de triméthylammonium dépendants du pH de copolymères d'acide acrylique et d'acide méthacrylique ; les copolymères dépendants du pH d'acide méthacrylique et d'acrylate de méthyle ; les copolymères dépendants du pH d'acide méthacrylique et de méthacrylate de méthyle ; les copolymères dépendants du pH d'acrylate d'éthyle et de méthacrylate de méthyle ; et les copolymères dépendants du pH de méthacrylate de butyle, de méthacrylate de diméthylaminoéthyle et de méthacrylate de méthyle, selon lequel :
(i) on mélange ces composants ensemble dans un solvant organique choisi parmi l'acétone, le méthanol, l'éthanol, l'isopropanol, le chlorure de méthylène, le chloroforme et les mélanges de ceux-ci, le solvant étant présent selon une quantité suffisante pour dissoudre complètement tant le premier que le deuxième composants, et le rapport de la quantité du premier composant à la quantité du deuxième composant étant de 10 à 2 000 parties en poids du deuxième composant pour 100 parties en poids du premier composant, et
(ii) on élimine ensuite le solvant pour obtenir ladite composition, le premier composant étant contenu sous une forme à l'état amorphe.

5. Procédé selon la revendication 4, dans lequel le rapport de la quantité du premier composant à la quantité du deuxième composant, est de 30 à 1 000 parties en poids du deuxième composant pour 100 parties en poids du premier composant.

6. Procédé selon la revendication 4, dans lequel le solvant est un mélange 3:7 d'éthanol et de chlorure de méthylène.

7. Utilisation d'une composition pharmaceutique selon les revendications 1 à 3, pour préparer un médicament.

8. Utilisation d'une composition pharmaceutique selon les revendications 1 à 3, pour préparer un agent anti-hypertensif.

## Revendications (Revendications pour l'(les) Etat(s) contractant(s) suivant(s): ES)

1. Procédé de préparation d'une composition pharmaceutique hautement absorbable comprenant du 1,4-dihydro-2,6-diméthyl-4-(3-nitrophényl)pyridine-3,5-dicarboxylate de méthyle et de 3-phényl-2-(E)-propényle en tant que son premier composant, et en tant que son deuxième composant, un copolymère dépendant du pH d'acide méthacrylique choisi parmi les sels de triméthylammonium dépendants du pH de copolymères d'acide acrylique et d'acide méthacrylique ; les copolymères dépendants du pH d'acide méthacrylique et d'acrylate de méthyle ; les copolymères dépendants du pH d'acide méthacrylique et de méthacrylate de méthyle ; les copolymères dépendants du pH d'acrylate d'éthyle et de méthacrylate de méthyle ; et les copolymères dépendants du pH de méthacrylate de butyle, de méthacrylate de diméthylaminoéthyle et de méthacrylate de méthyle, selon lequel (i) on mélange ces composants ensemble dans un solvant organique choisi parmi l'acétone, le méthanol, l'éthanol, l'isopropanol, le chlorure de méthylène, le chloroforme et les mélanges de ceux-ci, ce solvant étant présent selon une quantité suffisante pour dissoudre complètement les deux composants, et le rapport de la quantité du premier composant à la quantité du deuxième composant, étant de 10 à 2 000 parties en poids du deuxième composant pour 100 parties en poids du premier composant, et (ii) on élimine ensuite le solvant pour obtenir la composition, le premier composant étant contenu sous une forme à l'état amorphe.

2. Procédé selon la revendication 1, dans lequel le rapport de la quantité du premier composant à la quantité du deuxième composant est de 30 à 1 000 parties en poids du deuxième composant pour 100 parties en poids du premier composant.

3. Procédé selon la revendication 1, dans lequel le solvant est un mélange 3:7 d'éthanol et de chlorure de méthylène.

4. Utilisation d'une composition pharmaceutique selon les revendications 1 à 3 pour préparer un médicament.

5. Utilisation d'une composition pharmaceutique selon les revendications 1 à 3 pour préparer un agent anti-hypertensif.
